# EUROPEAN PATENT APPLICATION

(11) **EP 0 816 316 A1**
(43) Date of publication of application: **07.01.1998**
(21) Application number: 96905035.0
(22) Date of filing: 08.03.1996
(51) Int. Cl.: C07C 31/20, C07C 29/136

(54) **PROCESS FOR PRODUCING 1,2-ETHANEDIOL DERIVATIVES**

(30) Priority: 10.03.1995 JP 78437/95; 31.03.1995 JP 97493/95
(71) Applicant: NITTO CHEMICAL INDUSTRY CO., LTD., Tokyo 100 (JP)
(72) Inventor: KOBAYASHI, Yoshimasa, Nitto Kagaku Kogyo K. K., Yokohama-shi, Kanagawa 230 (JP); MORI, Hiroyuki, Nitto Kagaku Kogyo K. K., Yokohama-shi, Kanagawa 230 (JP); MORITA, Hiraki, Nitto Kagaku Kogyo K. K., Yokohama-shi, Kanagawa 230 (JP)
(74) Representative: Hansen, Bernd, Dr. Dipl.-Chem.
(86) International application number: JP9600571
(87) International publication number: WO9628405

(57) **Abstract**

A process for preparing a 1,2-ethanediol derivative in a high yield by reducing an α-hydroxy carboxylic acid ester derivative in a protic solvent or a hydrous aprotic polar solvent in the presence of a sodium boron hydride compound. A process for preparing a 1,2-ethanediol derivative in a high yield by esterifying an α-hydroxy carboxylic acid derivative to synthesize an α-hydroxy carboxylic acid ester derivative, and reducing this ester derivative in a protic solvent or a hydrous aprotic polar solvent in the presence of a sodium borohydride compound in the same reaction vessel.

## Description

### TECHNICAL FIELD

The present invention relates to a process for preparing 1,2-ethanediol derivatives useful as intermediates for the pharmaceuticals and agricultural chemicals, which derivatives are represented by the formula (1): 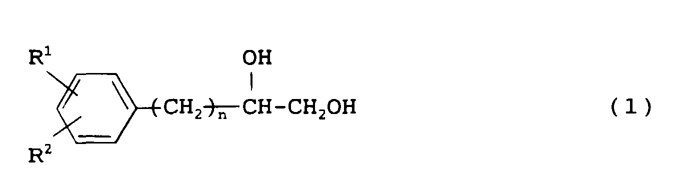 wherein R¹ and R² may be identical or different and represent independently a hydrogen atom, a C₁-C₄ linear or branched alkyl group which may be substituted with a halogen, a C₁-C₄ linear or branched alkoxyl group which may be substituted with a halogen, a hydroxyl group, an amino group which may have a substituent, a nitro group or a halogen atom, or R¹ and R² may be combined to form a lower alkylenedioxy group; and n is an integer of 0 to 3.

### BACKGROUND ART

For preparation of 1,2-ethanediol derivatives, such techniques as reducing an α-hydroxy carboxylic acid ester derivative with lithium aluminum hydride or reducing an α-hydroxy carboxylic acid derivative with diborane or a boran/dimethyl sulfide complex have been well known in the art.

For instance, for preparation of 1-phenyl-1,2-ethanediol, there are known a method comprising reduction of a mandelic acid methyl ester with lithium aluminum hydride (J. Amer. Chem. Soc., Vol. 81, p. 6456, (1959)) and a method comprising reduction of mandelic acid with diborane (mentioned as Referential Example in US-A-4,391,826). Also, for preparation of 1-(3-chlorophenyl)-1,2-ethanediol, a method comprising reduction of 3-chloromandelic acid with a borane/dimethyl sulfide complex (mentioned as Referential Example in EP-A-28,105) is known.

However, since lithium aluminum hydride is subject to hydrolysis in water, it is essential to use an anhydrous solvent in these methods. Also, as lithium aluminum hydride is a strong reducing agent and liable to induce a side reaction when used for reduction of α-hydroxy carboxylic acid derivatives, it is necessary to protect the hydroxyl groups prior to the reducing reaction. Thus, because of its lack of operational safety and reaction stability and also because of complexity of the reaction process, lithium aluminum hydride is generally unsuited for use in the industrial preparation processes.

Diborane and borane/dimethyl sulfide complexes are used by preference in laboratories as they are capable of specifically reducing the carboxyl groups alone without affecting the other functional groups. However, these borane-based reducing agents are unstable in water and have a nature to ignite in the air or in water, so that it is necessary to use an anhydrous solvent for the reducing reaction. Further, the borane-based reducing agents are expensive and need to be used in comparatively large quantities for a reducing reaction, so that the process using such a reducing agent is disadvantageous economically. Further, the borane-based reducing agents have strong toxicity to the human body, and moreover, in case of using a borane/dimethyl sulfide complex, the sanitary problems tend to take place because of the nasty smell of dimethyl sulfide which is a constituent of the reducing agent. For these reasons, the borane-based reducing agents are generally unsuited for use in the industrial preparation processes.

Thus, because of these restrictions on safety and economy, there is presently available no better substance than sodium borohydride for industrial use as reducing agent.

Sodium borohydride is popularly used as a selective reducing agent for the carbonyl compounds such as aldehydes and ketones and the carboxylic acid chlorides, but it is known self-evidently that this compound does not work well as a reducing agent for the carboxylic acid esters.

For instance, an attempt for reducing a mandelic acid ethyl ester with sodium borohydride in anhydrous dioxane is disclosed in the afore-mentioned literature (J. Amer. Chem. Soc., Vol. 81, p. 6456, (1959)), but it is stated that the reaction did not proceed at all and the attempt has ended up in merely recovering the starting material. Also, when a simple aliphatic ester is reduced with sodium borohydride in a protonic solvent, the reaction rate is very low and this operation is impractical from the industrial viewpoint. It is also reported that when ethyl laurate was reduced with tetrabutylammonium boron hydride in an aprotic solvent such as dichloromethane for 4 days, the reaction proceeded only 25% (J. Org. Chem., Vol. 41, p. 690, (1976)). Only as an exception, there is a report that a certain type of ester could be reduced to a corresponding alcohol by using 5 to 20 times as much amount of sodium borohydride as the ester by mole (J. Org. Chem., Vol. 28, p. 3261, (1963)). It can be learned from this report that a large amount of a reducing agent is required in a reducing reaction using sodium boron hydride.

Thus, no attempt has ever been made for reducing carboxylic acid esters to the corresponding alcohols by using a moderate amount of sodium borohydride, or such attempts, if any, have ended in failure.

Especially, the present inventors have not yet been informed of any report that the α-hydroxy carboxylic acid ester derivatives could be easily reduced to the corresponding 1,2-ethanediol derivatives by using sodium borohydride.

1,2-ethanediol derivatives are useful as intermediates for the pharmaceuticals and agricultural chemicals, but their preparation process involves many problems such as mentioned above. Development of a more simple and economical industrial preparation process of 1,2-ethanediol derivatives has therefore been desired.

### DISCLOSURE OF THE INVENTION

In order to solve these problems, the present inventors have made extensive researches on the reducing reactions using the sodium borohydride compounds, particularly concerning the amount of the reducing agent used and the reaction solvents, and as a result found quite surprisingly that the α-hydroxy carboxylic acid ester derivatives can be easily reduced in a medium containing at least one solvent selected from protic solvents and hydrous aprotic polar solvents in the presence of a sodium borohydride compound, and the objective 1,2-ethanediol derivative can be produced safely and economically. The present invention has been attained on the basis of this finding.

Thus, the present invention relates to a process for preparing 1,2-ethanediol derivatives represented by the formula (1) shown above, which comprises reducing an α-hydroxy carboxylic acid ester derivative represented by the formula (2): 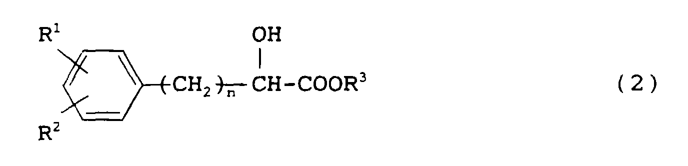 (wherein R¹ and R² may be identical or different and represent independently a hydrogen atom, a C₁-C₄ linear or branched alkyl group which may be substituted with a halogen, a C₁-C₄ linear or branched alkoxyl group which may be substituted with a halogen, a hydroxyl group, an amino group which may have a substituent, a nitro group or a halogen atom, or R¹ and R² may be combined to form a lower alkylenedioxy group; R³ represents a C₁-C₄ linear or branched alkyl group; and n is an integer of 0 to 3) in a solvent medium containing at least one solvent selected from protic solvents and hydrous aprotic polar solvents in the presence of a sodium borohydride compound.

The present invention also relates to a process for preparing 1,2-ethanediol derivatives represented by the formula (1) shown above, which comprises esterifying an α-hydroxy carboxylic acid derivative represented by the formula (3): 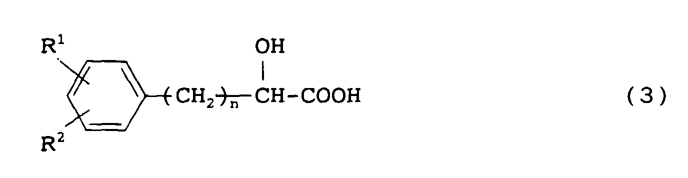 (wherein R¹, R² and n are as defined above) to synthesize an α-hydroxy carboxylic acid ester derivative represented by the formula (2): 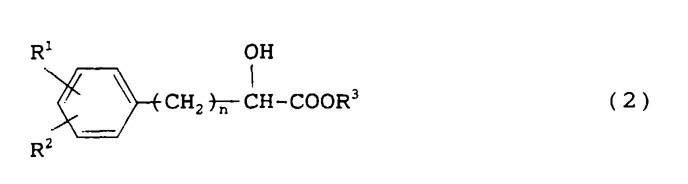 (wherein R¹, R², R³ and n are as defined above), adding a sodium borohydride compound to the resulting reaction solution without isolating said α-hydroxy carboxylic acid ester derivative, and reducing said α-hydroxy carboxylic acid ester derivative in a solvent containing at least one selected from protic solvents and hydrous aprotic polar solvents.

### BEST MODE FOR CARRYING OUT THE INVENTION

The α-hydroxy carboxylic acid derivatives usable as starting material in the present invention are represented by the formula (3) shown above. The C₁-C₄ linear or branched alkyl groups which may be substituted with a halogen, represented by R¹ and R² in the formula (3), include methyl, ethyl, n-propyl, isopropyl, n-butyl, isobutyl, sec-butyl, tert-butyl, trifluoromethyl, trichloromethyl, etc. The C₁-C₄ linear or branched alkoxy groups which may be substituted with a halogen include methoxy, ethoxy, n-propoxy, isopropoxy, n-butoxy, sec-butoxy, tert-butoxy, trifluoromethoxy, trichloromethoxy, etc. The amino groups which may have a substituent include amino, methylamino, ethylamino, isopropylamino, dimethylamino, diethylamino, methylethylamino, morphorino, pyridino, piperidino, pyrrole group, imidazole group, triazole group, etc. The halogen atoms include fluorine atom, chlorine atom, bromine atom and iodine atom. The lower alkylenedioxy groups formed by combination of R¹ and R² include methlenedioxy, ethylenedioxy, trimethylenedioxy, etc. n represents an integer of 0 to 3.

Examples of the α-hydroxy acid derivatives defined above include mandelic acid, 2-chloromandelic acid, 3-chloromandelic acid, 4-chloromandelic acid, 2-methylmandelic acid, 3-methylmandelic acid, 4-methylmandelic acid, 2-hydroxymandelic acid, 3-hydroxymandelic acid, 4-hydroxymandelic acid, 2-methoxymandelic acid, 3-methoxymandelic acid, 4-methoxymandelic acid, 4-trifluoromethylmandelic acid, 2-aminomandelic acid, 3-aminomandelic acid, 4-aminomandelic acid, 2-nitromandelic acid, 3-nitromandelic acid, 4-nitromandelic acid, 2,4-dichloromandelic acid, 2,4-difluoromandelic acid, 3,4-methylenedioxymandelic acid, phenyllactic acid, 4-hydroxyphenyllactic acid, 4-aminophenyllactic acid, 4-nitrophenyllactic acid, 3-chlorophenyllactic acid, 4-methoxyphenyllactic acid, 2,4-dichlorophenyllactic acid, 2,4-difluorophenyllactic acid, 4-phenyl-2-hydroxybutyric acid, 4-(4-aminophenyl)-2-hydroxybutyric acid, 4-(4-hydroxyphenyl)-2-hydroxybutyric acid, 4-(4-nitrophenyl)-2-hydroxybutyric acid, 4-(3-chlorophenyl)-2-hydroxybutyric acid, 4-(2,4-difluorophenyl)-2-hydroxybutyric acid, 5-phenyl-2-hydroxypentanoic acid, 5-(4-aminophenyl)-2-hydroxypentanoic acid, 5-(4-hydroxyphenyl)-2-hydroxypentanoic acid, 5-(3-chlorophenyl)-2-hydroxypentanoic acid, and 5-(2,4-difluorophenyl)-2-hydroxypentanoic acid, etc.

These α-hydroxy carboxylic acid derivatives may be either racemic modification or optically active compounds including R-form and S-form. An optically active compound may be properly selected in consideration of bioactivity of the pharmaceutical or agricultural chemical produced from a 1,2-ethanediol derivative.

An α-hydroxy carboxylic acid derivative usable as starting material in the present invention can be easily obtained by reacting a corresponding aldehyde derivative with HCN and then hydrolyzing the reaction product.

An optically active α-hydroxy carboxylic acid derivative can be produced by the known methods, such as reacting a corresponding aldehyde derivative with HCN and then enzymatically hydrolyzing the resulting cyanohydrin derivative, or separating an optically active compound from a racemic α-hydroxy carboxylic acid derivative by an ordinary technique of optical resolution, or subjecting a corresponding α-keto-acid to enzymatic asymmetric reduction.

The α-hydroxy carboxylic acid ester derivatives usable as starting material in the present invention are represented by the formula (2). In the formula (2), R¹, R² and n are as defined above, and R³ represents a C₁-C₄ linear or branched alkyl group, which includes methyl, ethyl, n-propyl, isopropyl, n-butyl, isobutyl, sec-butyl, tert-butyl, etc.

The α-hydroxy carboxylic acid ester derivative defined above can be produced by esterifying a corresponding α-hydroxy carboxylic acid derivative by a known method. For example, the ester derivative can be easily produced by adding a methylating agent such as diazomethane or dimethylsulfuric acid to the α-hydroxy carboxylic acid derivative, or reacting the α-hydroxy carboxylic acid derivative in an alcohol such as methanol, ethanol, propanol,isopropanol, n-butanol, isobutanol, sec-butanol, tert-butanol or the like in the presence of an acid catalyst.

The acid catalysts usable here include sulfuric acid, hydrochloric acid, nitric acid, p-toluenesulfonic acid and methanesulfonic acid, etc.

Examples of the α-hydroxy carboxylic acid ester derivatives that can be produced in the manner described above include the esters corresponding to the α-hydroxy carboxylic acid derivatives whose examples were mentioned above. These α-hydroxy carboxylic acid ester derivatives may be either racemic modifications or optically active compounds including R-form and S-form. An optically active compound may be properly selected in consideration of bioactivity of the pharmaceutical or agricultural chemical preparation produced from a 1,2-ethanediol derivative.

In the process of the present invention, the reducing reaction proceeds while maintaining the optical activity of the starting material α-hydroxy carboxylic acid derivative or α-hydroxy carboxylic acid ester derivative, so that it is possible to obtain an objective 1,2-ethanediol derivative having a desired optical activity credited to the starting material.

In production of an optically active 1,2-ethanediol derivative by reducing a carboxylic acid having an optical activity, only use of a borane-based reducing agent such as borane or a borane/dimethyl sulfide complex has been reported (aforementioned US-A-4,391,826 and EP-A-28,105), and no disclosure has ever been made on use of sodium borohydride, as far as the present inventors know.

The reducing agents suited for use in the present invention are sodium borohydride compounds such as sodium borohydride, sodium cyanoborohydride and sodium alkoxyborohydride. Sodium borohydride is preferred.

The amount of a sodium borohydride compound used in the present invention is 0.75 to 4 times, preferably one to 3 times the mole of the α-hydroxy carboxylic acid ester derivative.

As the solvent in the process of the present invention, a protic solvent and/or a hydrous aprotic polar solvent can be used.

As the protic solvent, water and/or a lower aliphatic alcohol such as methanol, ethanol, propanol, isopropanol, n-butanol, sec-butanol, tert-butanol or the like are preferably used. It is also possible to use a mixed solvent system prepared by adding water to a lower aliphatic alcohol.

As the aprotic polar solvent, tetrahydrofuran, dimethylformamide, dioxane, dimethyl sulfoxide, dimethoxyethane and the like can be used, but a hydrous aprotic polar solvent in which water has been added as proton source is preferred.

It is also possible to use a combination of a protic solvent and a hydrous aprotic polar solvent, each mentioned above.

In the process of the present invention, the reaction temperature is in a range of -50°C to the boiling point of the solvent used, preferably from -20°C to 50°C. The reaction time varies depending principally on the reaction temperature, the starting compounds and the type of the solvent used, but usually it is within 12 hours, preferably in the range of 10 minutes to 7 hours.

The end product 1,2-ethanediol derivative is collected from the reaction mixture by a conventional method after the reaction is completed. For instance, an organic solvent immiscible with water is added to the reaction mixture, and after washing with water, the solvent is evaporated away to obtain the objective product. If necessary, the obtained 1,2-ethanediol derivative may be further purified by a conventional method such as recrystallization, reprecipitation, distillation or chromatography.

In another embodiment of the present invention, an α-hydroxy carboxylic acid derivative of the formula (3) is esterified to form an α-hydroxy carboxylic acid ester derivative of the formula (2), and with this α-hydroxy carboxylic acid ester derivative being left unisolated, a sodium boron hydride compound is added to the reaction solution to reduce it, thereby producing 1,2-ethanediol. This embodiment of the present invention is discussed below.

For esterifying the α-hydroxy carboxylic acid derivative in the above process, the known esterification methods can be used, but it is advisable to employ a method in which said derivative is esterified in an aliphatic alcohol solvent by using an acid catalyst. As the acid catalyst, hydrochloric acid, sulfuric acid, nitric acid, p-toluenesulfonic acid, methanesulfonic acid and the like can be favorably used. The amount of the acid catalyst used is usually in a range of 1/2 to 1/1,000 parts by weight, preferably from 1/5 to 1/200 parts by weight, per one part by weight of the α-hydroxy carboxylic acid derivative. The esterification reaction is carried out at a temperature in a range of 0°C to the boiling point of the solvent. As the aliphatic alcohol used as solvent, methanol, ethanol, propanol, isopropanol, n-butanol, sec-butanol, tert-butanol, etc., can be used, but it is preferred to use an aliphatic alcohol corresponding to the alcohol moiety of the ester.

In the above process, there is no need of isolating the α-hydroxy carboxylic acid ester derivative from the resulting reaction solution, and it suffices to neutralize the reaction solution with an alkali as desired. The alkali neutralizing agent used here is not specified, but an aqueous sodium hydroxide solution, an aqueous potassium hydroxide solution, an aqueous sodium carbonate solution or an aqueous sodium hydrogencarbonate soluton is preferably used. Such a neutralizing agent has only to be added in an amount sufficient to neutralize the acid catalyst used.

If necessary, after the reaction solution is neutralized with an alkali, a sodium borohydride compound is added to the reaction solution containing the α-hydroxy carboxylic acid ester derivative in the same reaction vessel to reduce the derivative.

As the sodium borohydride compound, sodium borohydride, sodium cyanoborohydride, sodium alkoxyborohydride and the like can be used, but sodium borohydride is preferred. The amound of the sodium borohydride compound used is 0.75 to 4 times, preferably one to 3 times the mole of the α-hydroxy carboxylic acid derivative as starting material.

As the solvent, there can be used the same solvent as employed for the esterification, for example, lower aliphatic alcohols such as methanol, ethanol, isopropanol, n-butanol, sec-butanol and tert-butanol. Other lower aliphatic alcohols or aprotic polar solvents, or mixtures thereof, are also usable. Although water used for neutralization is already present in the system, water may be further added to the reaction solvent.

The reaction temperature is in a range of -50°C to the boiling point of the solvent used, preferably from -20°C to 50°C. The reaction time is variable depending principally on the reaction temperature, the starting compounds and the type of the solvent used, but it is usually within 12 hours, preferably in a range of 10 minutes to 7 hours.

The end product 1,2-ethanediol derivative is collected from the reaction mixture by a conventional method after the reaction is completed. For instance, an organic solvent immiscible with water is added to the reaction mixture, and after washing with water, the solvent is evaporated away to obtain the objective product. If necessary, the obtained 1,2-ethanediol derivative may be purified by a conventional method such as recrystallization, reprecipitation, distillation or chromatography.

It has been found that when the above reaction is carried out using an optically active α-hydroxy carboxylic acid derivative or α-hydroxy carboxylic acid ester derivative as the starting compound, the obtained 1,2-ethanediol derivative maintains a corresponding steric configuration. It has thus become possible to obtain a corresponding optically active 1,2-ethanediol derivative having a steric configuration from an optically active α-hydroxy carboxylic acid derivative or α-hydroxy carboxylic acid ester derivative. Optical purity of the obtained optically active 1,2-ethanediol derivative was measured by high-performance liquid chromatography using an optically resolving column.

The present invention is explained in more detail with reference to the following examples, but it should not be construed that the scope of the present invention is restricted by these examples.

### Example 1

5 g of methyl mandelate was dissolved in 10 ml of methanol, to which 1.25 g of sodium borohydride (1.1 time methyl mandelate by mole) was added under ice cooling, and the reaction solution was stirred at room temperature (about 20°C) for 2 hours. Then the reaction solution was neutralized with 2N hydrochloric acid, and after the most part of the solvent methanol was evaporated away, water was added to the reaction solution followed by extraction with chloroform. The obtained chloroform layer was dried over magnesium sulfate and filtered. The filtrate was concentrated under reduced pressure to obtain 4.5 g of a colorless oil. Recrystallization of this substance gave 3.8 g of 1-phenyl-1,2-ethandiol as colorless crystals in a 92% yield, mp 67-68°C.

### Example 2

The same procedure as in Example 1 was repeated except that sodium caynoborohydride was used as the reducing agent, to obtain 1-phenyl-1,2-ethanediol in an 89% yield.

### Examples 3-10 and Comparative Examples 1-3

The same procedure as in Example 1 was repeated except for use of the solvents shown in Table 1. The yields of the product 1-phenyl-1,2-ethanediol are shown in Table 1.

**Table 1**

| Example and Comp. Example | | Solvent | Yield (%) |
|---|---|---|---|
| Example | 3 | Methanol | 91 |
| | 4 | Methanol +water (2 ml) | 93 |
| | 5 | Ethanol | 92 |
| | 6 | Isopropanol | 80 |
| | 7 | Tetrahydrofuran + water (2 ml) | 89 |
| | 8 | Dioxane + water (2 ml) | 46 |
| | 9 | Dimethylformamide + water (2 ml) | 77 |
| | 10 | Dimethyl sulfoxide + water (2 ml) | 70 |
| Comp. Example | 1 | Tetrahydrofuran | 41 |
| | 2 | Dioxane | 13 |
| | 3 | Dimethylformamide | 64 |

### Examples 11-31

The same procedure as in Example 1 was repeated except that the type of the starting material α-hydroxy carboxylic acid ester derivative and the molar ratio of the sodium borohydride catalyst to the α-hydroxy carboxylic acid ester derivative were changed as shown in Table 2, to obtain the corresponding 1,2-ethanediol derivatives in the yields shown in Table 2.

**Table 2**

| Example | α-hydroxy carboxylic acid ester derivative | Times by mole of catalyst | Yield (%) |
|---|---|---|---|
| 11 | Ethyl mandelate | 1.1 | 92 |
| 12 | Isopropyl mandelate | 1.1 | 48 |
| 13 | t-Butyl mandelate | 1.1 | 27 |
| 14 | Methyl 2-chloromandelate | 1.1 | 88 |
| 15 | Methyl 3-chloromandelate | 1.1 | 94 |
| 16 | Methyl 4-chloromandelate | 1.1 | 86 |
| 17 | Methyl 4-hydroxymandelate | 1.1 | 76 |
| 18 | Methyl 4-methoxymandelate | 1.1 | 84 |
| 19 | Methyl 2,4-difluoromandelate | 1.1 | 90 |
| 20 | Methyl 2,4-dichloromandelate | 1.1 | 89 |
| 21 | Methyl 4-aminomandelate | 1.1 | 78 |
| 22 | Methyl 4-nitromandelate | 1.1 | 75 |
| 23 | Methyl 4-methylmandelate | 1.1 | 91 |
| 24 | Methyl 4-trifluoromethylmandelate | 1.1 | 86 |
| 25 | Methyl phenyllactate | 2.0 | 85 |
| 26 | Methyl 4-hydroxyphenyllactate | 2.0 | 76 |
| 27 | Methyl 3-chlorophenyllactate | 2.0 | 78 |
| 28 | Methyl 4-methoxyphenyllactate | 2.0 | 82 |
| 29 | Methyl 4-aminophenyllactate | 2.0 | 67 |
| 30 | Ethyl 4-phenyl-2-hydroxybutyrate | 2.5 | 72 |
| 31 | Methyl 5-phenyl-2-hydroxypentanoate | 2.5 | 62 |

### Example 32

0.2 g of concentrated sulfuric acid was added to a solution of 5 g of mandelic acid in 10 ml of methanol, and the reaction solution was heated at the refluxing temperature for 3 hours for esterification. The esterified reaction solution was cooled to room temperature and neutralized with a 2N aqueous sodium hydroxide solution. 1.25 g of sodium borohydride (1.0 time the mandelic acid by mole) was added to the reaction solution under ice cooling and then stirred at room temperature (about 20°C) for 2 hours. Thereafter, the reaction solution was neutralized with 2N hydrochloric acid, and after the most part of the solvent methanol was evaporated away, water was added to the reaction solution followed by extraction with chloroform. The obtained chloroform layer was dried over magnesium sulfate and filtered. The filtrate was concentrated under reduced pressure to obtain 4.9 g of a colorless oil. Recrystallization of this substance gave 3.5 g (85% yield) of 1-phenyl-1,2-ethanediol as colorless crystals, mp 66-68°C.

### Example 33

The same procedure as in Example 32 was repeated except that 3-chloromandelic acid was used as starting material, to obtain 3.6 g of 1-(3-chlorophenyl)-1,2-ethanediol as colorless crystals.

### Example 34

The same procedure as in Example 32 was repeated except that 4-hydroxyphenyllactic acid was used as starting material, to obtain 3.5 g of 1-(4-hydroxybenzyl)-1,2-ethanediol as colorless crystals.

### Example 35

The same procedure as in Example 32 was repeated except that R-form mandelic acid with an optical purity of 100% ee was used as starting material, to obtain 3.4 g of R-form 1-phenyl-1,2-ethanediol as colorless crystals. Optical purity of this substance measured by HPLC using an optically resolving column was 99% ee.

### Example 36

The same procedure as in Example 32 was repeated out except that R-form 3-chloromandelic acid with 98% ee optical purity was used as starting material, to obtain 3.3 g of R-form 1-(3-chlorophenyl)-1,2-ethanediol as colorless crystals. Optical purity of this substance measured by HPLC using an optically resolving column was 97% ee.

### Example 37

The same procedure of Example 32 was repeated except that S-form mandelic acid with 95% ee optical purity was used as starting material, to obtain 3.4 g of S-form 1-phenyl-1,2-ethanediol as colorless crystals. Optical purity of this substance measured by HPLC on an optically resolving column was 95% ee.

### INDUSTRIAL APPLICABILITY

According to the present invention, it is possible to produce 1,2-ethanediol derivatives from α-hydroxy carboxylic acid ester derivatives by using as reducing agent a sodium borohydride compound which is inexpensive and safe, so that the present invention can be utilized for simple and economically advantageous industrial production of intermediates for the pharmaceuticals and agricultural chemicals.

## Claims

1. A process for preparing a 1,2-ethanediol derivative represented by the formula (1): 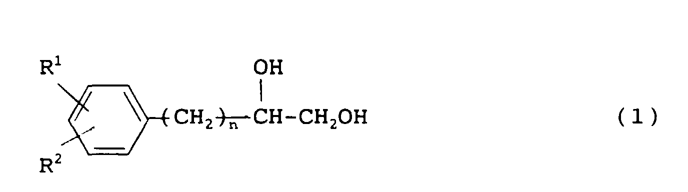 (wherein R¹ and R² may be identical or different and represent independently a hydrogen atom, a C₁-C₄ linear or branched alkyl group which may be substituted with a halogen, a C₁-C₄ linear or branched alkoxy group which may be substituted with a halogen, a hydroxyl group, an amino group which may have a substituent, a nitro group or a halogen atom, or R¹ and R² may be combined to form a lower alkylenedioxy group; and n is an integer of 0 to 3) which comprises reducing an α-hydroxy carboxylic acid ester derivative represented by the formula (2): 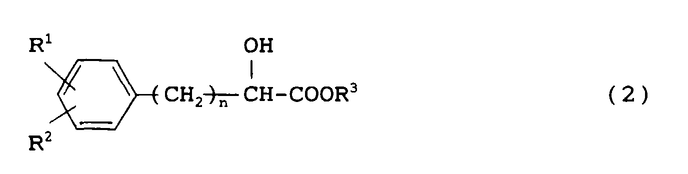 (wherein R¹, R² and n are as defined above, and R³ represents a C₁-C₄ linear or branched alkyl group) in a solvent containing at least one selected from protic solvents and hydrous aprotic polar solvents in the presence of a sodium borohydride compound.

2. A process for preparing a 1,2-ethanediol derivative represented by the formula (1): 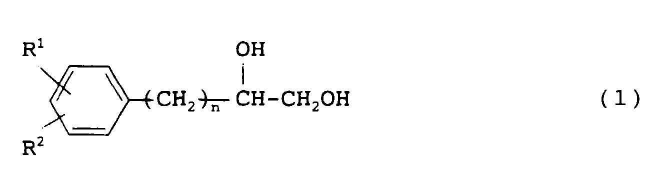 (wherein R¹ and R² may be identical or different and represent independently a hydrogen atom, a C₁-C₄ linear or branched alkyl group which may be substituted with a halogen, a C₁-C₄ linear or branched alkoxy group which may be substituted with a halogen, a hydroxyl group, an amino group which may have a substituent, a nitro group or a halogen atom, or R1 and R2 may be combined to form a lower alkylenedioxy group; and n is an integer of 0 to 3) which comprises esterifying an α-hydroxy carboxylic acid derivative represented by the formula (3): 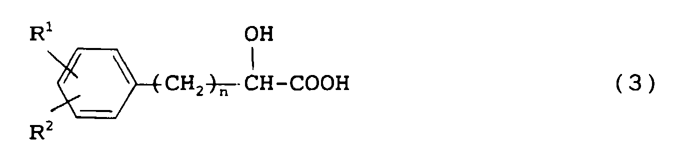 (wherein R¹, R² and n are as defined above) to synthesize an α-hydroxy carboxylic acid ester derivative represented by the formula (2): 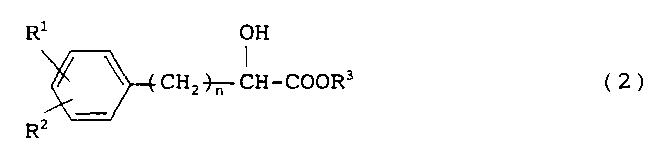 (wherein R¹, R² and n are as defined above, and R³ represents a C₁-C₄ linear or branched alkyl group), adding a sodium borohydride compound to the resulting reaction solution without isolating the synthesized α-hydroxy carboxylic acid ester derivative, and reducing said α-hydroxy carboxylic acid ester derivative in a solvent containing at least one selected from protic solvents and hydrous aprotic polar
solvents.

3. The process according to Claim 1 or 2, wherein the protic solvent is water and/or a lower aliphatic alcohol, and the aprotic polar solvent is at least one member selected from the group consisting of tetrahydrofuran, dimethylformamide, dioxane, dimethyl sulfoxide and dimethoxyethane.

4. The process according to Claim 1 or 2, wherein the protic solvent is a lower aliphatic alcohol which may contain water.

5. The process according to Claim 1, wherein the amount of the sodium borohydride compound used is 0.75 to 4 times the mole of the α-hydroxy carboxylic acid ester derivative of the formula (2).

6. The process according to Claim 2, wherein the amount of the sodium borohydride compound used is 0.75 to 4 times the mole of the α-hydroxy carboxylic acid derivative of the formula (3).

7. The process according to Claim 2, wherein an acid catalyst is used for the esterification.

8. The process according to Claim 1, wherein the α-hydroxy carboxylic acid ester derivative of the formula (2) is an optically active compound, and a corresponding 1,2-ethanediol derivative is obtained maintaining its steric structure.

9. The process according to Claim 2, wherein the α-hydroxy carboxylic acid ester derivative of the formula (3) is an optically active compound, and a corresponding 1,2-ethanediol derivative is obtained maintaining its steric structure.

10. The process according to Claim 1 or 2, wherein the sodium borohydride compound is sodium borohydride.

11. Use of a sodium borohydride compound as a reducing agent in a reaction for reducing an α-hydroxy carboxylic acid ester derivative represented by the formula (2): 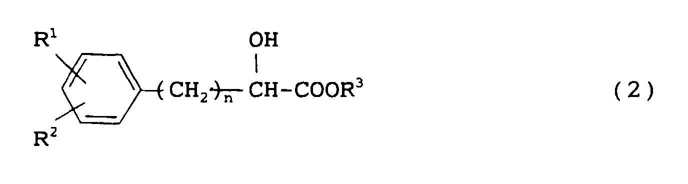 (wherein R¹ and R² may be identical or different and represent independently a hydrogen atom, a C₁-C₄ linear or branched alkyl group which may be substituted with a halogen, a C₁-C₄ linear or branched alkoxy group which may be substituted with a halogen, a hydroxyl group, an amino group which may have a substituent, a nitro group or a halogen atom, or R¹ and R² may be combined to form a lower alkylenedioxy group; R³ represents a C₁-C₄ linear or branched alkyl group; and n is an integer of 0 to 3) to a 1,2-ethanediol derivative represented by the formula (1): 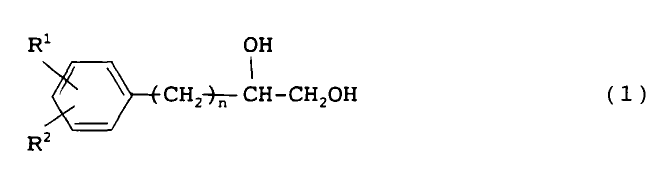 (wherein R¹, R² and n are as defined above).
